(19) Europäisches Patentamt
European Patent Office
Office européen des brevets

(11) Veröffentlichungsnummer: **0 099 523**
**A2**

# (12) EUROPÄISCHE PATENTANMELDUNG

(21) Anmeldenummer: 83106686.5

(22) Anmeldetag: 08.07.83

(51) Int. Cl.³: C 07 C 69/74
C 07 C 67/14, C 07 C 69/75
A 01 N 37/08

(30) Priorität: 21.07.82 DE 3227220

(43) Veröffentlichungstag der Anmeldung: 01.02.84 Patentblatt 84/5

(84) Benannte Vertragsstaaten: BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Maurer, Fritz, Dr.
Roeberstrasse 8
D-5600 Wuppertal 1(DE)

(72) Erfinder: Brandes, Wilhelm, Dr.
Eichendorffstrasse 3
D-5653 Leichlingen 1(DE)

(72) Erfinder: Reinecke, Paul, Dr.
Lessingstrasse 11
D-5090 Leverkusen 3(DE)

## (54) Jodpropargylcarbonsäureester.

(57) Die Erfindung betrifft neue Jodpropargylcarbonsäureester, ein Verfahren zu ihrer Herstellung sowie ihre Verwendung als Pflanzenschutzmittel.

Die neuen Verbindungen der Formel

$$I-C\equiv C-CH_2-O-CO-A-CO-O-CH_2-C\equiv C-I \quad (I)$$

in welcher

A die in der Beschreibung angegebene Bedeutung besitzt,

werden erhalten, wenn man Cycloakylenedicarbonsäuren zunächst mit Thionylchlorid umsetzt und die erhaltenen Säuredichloride in Gegenwart eines Säurebindemittels mit Iodpropargylalkohol umsetzt.

Als Pflanzenschutzmittel können die erfindungsgemäßen Wirkstoffe zur Bekämpfung von Venturia-Arten, von Leptosphaeria-Arten, von Oomyceten, von Mehltau- und Rostkrankheiten sowie zur Bekämpfung von Reiskrankheiten eingesetzt werden.

EP 0 099 523 A2

BAYER AKTIENGESELLSCHAFT 5090 Leverkusen, Bayerwerk
Zentralbereich
Patente, Marken und Lizenzen Bas/Slr/bo
Ia

## Jodpropargylcarbonsäureester

Die Erfindung betrifft neue Jodpropargylcarbonsäureester, ein Verfahren zu ihrer Herstellung sowie ihre Verwendung als Pflanzenschutzmittel.

Es ist bereits bekannt geworden, daß Verbindungen wie z.B. Zinkethylen-1,2-bis-(dithiocarbamat) oder N-Trichlormethylthio-tetrahydrophthalimid fungizide Eigenschaften aufweisen. (Vergleiche z.B. R.Wegler, 'Chemie der Pflanzenschutz- und Schädlingsbekämpfungsmittel', Springer Verlag, Berlin 1970, Band 2). Deren Wirkung ist jedoch, insbesondere bei niedrigen Aufwandmengen und -konzentrationen, nicht immer voll befriedigend.

Es wurden neue Jodpropargylcarbonsäureester der allgemeinen Formel (I) :

$$I-C{\equiv}C-CH_2-O-CO-A-CO-O-CH_2-C{\equiv}C-I \qquad (I)$$

in welcher

A für einen gegebenenfalls substituierten zweifach verknüpften Cycloalkylenrest steht,

gefunden.

Je nach Art der Verknüpfung des Cycloalkylenrestes A können die Verbindungen der Formel (I) in zwei geometrischen Isomerenformen (cis oder trans) vorliegen. Sowohl die Isomeren als auch die Gemische beider werden erfindungsgemäß beansprucht.

Weiterhin wurde gefunden, daß man die Jodpropargylester der allgemeinen Formel (I) :

$$I-C\equiv C-CH_2-O-CO-A-CO-O-CH_2-C\equiv C-I \qquad (I)$$

in welcher

A für einen gegebenenfalls substituierten zweifach verknüpften Cycloalkylenrest steht,

erhält, wenn man Cycloalkylendicarbonsäuren der allgemeinen Formel (II) :

$$HO-CO-A-CO-OH \qquad (II)$$

in welcher

A die oben angegebene Bedeutung hat,

zunächst mit Thionylchlorid, gegebenenfalls in Gegenwart eines Verdünnungsmittels, umsetzt (1. Stufe), und die so erhaltenen Cycloalkylendicarbonsäuredichloride der allgemeinen Formel (III) :

Cl-CO-A-CO-Cl (III)

in welcher

A die oben angegebene Bedeutung hat,

in Gegenwart eines Säurebindemittels und gegebenenfalls in Gegenwart eines Verdünnungsmittels mit Jodpropargylalkohol umsetzt (2. Stufe).

Die neuen Jodpropargylcarbonsäureester der Formel (I) weisen starke fungizide Eigenschaften auf. Dabei zeigen die erfindungsgemäßen Verbindungen der Formel (I) überraschenderweise eine bessere fungizide Wirksamkeit, als die nach dem Stand der Technik bekannten Stoffe Zink-ethylen-1,2-bis-(dithiocarbamat) und N-Trichlormethylthio-tetrahydrophthalimid. Die erfindungegemäßen Stoffe stellen somit eine Bereicherung der Technik dar.

Die erfindungsgemäßen Jodpropargylcarbonsäureester sind durch die Formel (I) allgemein definiert. Bevorzugt sind Stoffe der Formel (I), bei welchen

A für einen gegebenenfalls einfach oder mehrfach, gleich oder verschieden substituierten 2-fach verknüpften Cycloalkylenrest mit 3 bis 7 Kohlenstoffatomen steht, wobei als Substituenten infrage kommen : Alkyl mit 1 bis 4 Kohlenstoffatomen und Halogen, insbesondere Fluor, Chlor oder Brom.

Besonders bevorzugt sind Verbindungen der Formel (I), bei denen

A für einen 2-fach verknüpften Cycloalkylenrest mit 3 bis 7 Kohlenstoffatomen steht, welcher gegebenenfalls ein- bis dreifach, gleich oder verschieden durch Methyl, Ethyl und Chlor substituiert sein kann.

Im einzelnen seien die folgenden Verbindungen der Formel (I) genannt :

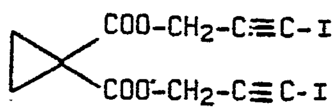

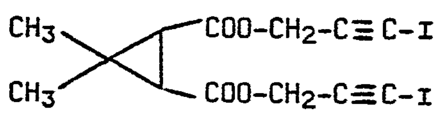

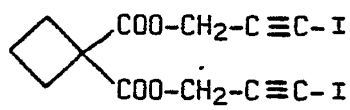

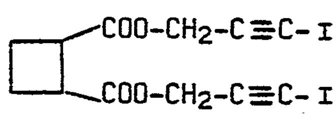

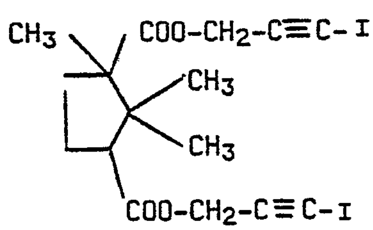

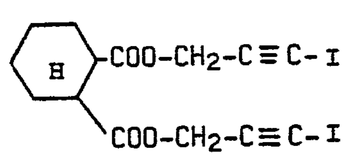

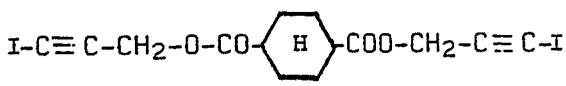

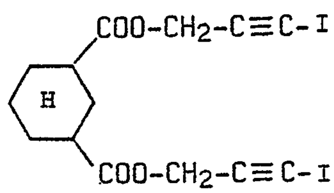

Verwendet man beispielsweise 3,3-Dimethyl-trans-1,2-cyclopropandicarbonsäure als Ausgangsprodukt, so kann der Reaktionsablauf des erfindungsgemäßen Verfahrens durch das folgende Formelschema wiedergegeben werden :

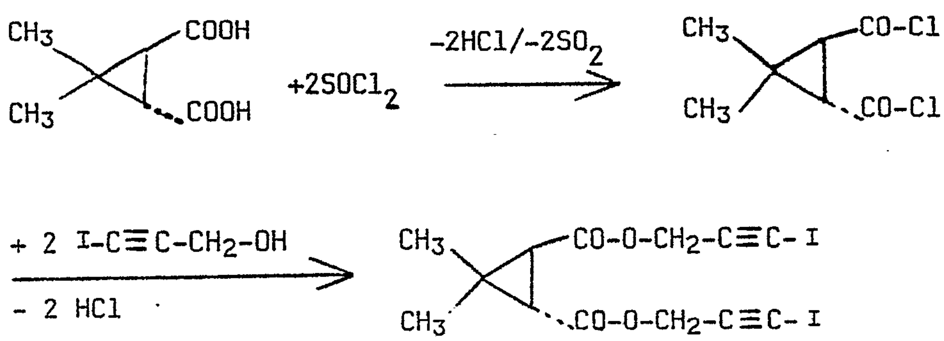


Die bei der Durchführung des erfindungsgemäßen Verfahrens als Ausgangsstoffe zu verwendenden Cycloalkylendicarbonsäuren sind durch die Formel (II) allgemein definiert. In dieser Formel steht

A vorzugsweise für diejenigen Reste, die bereits im Zusammenhang mit der Beschreibung der erfindungsgemäßen Stoffe der Formel (I) vorzugsweise für diese Gruppe genannt wurden.

Die Cycloalkylendicarbonsäuren der Formel (II) sind seit langer Zeit bekannt (vergleiche z.B. Houben-Weyl, 'Methoden der org. Chemie', Band 8, 4. Auflage, S. 359 ff, ferner Beilstein, Handbuch der organischen Chemie, H 9, 721 ff, System-Nummer 964).

Der außerdem zur Durchführung des erfindungsgemäßen Verfahrens benötigte Jodpropargylalkohol ist schon seit langer Zeit bekannt (vergleiche z.B. Lespieau, Ann.Chim. Phys. /7/, 11, 273 (1897)).

Für die erfindungsgemäße Umsetzung kommen als Verdünnungsmittel in der 1. und in der 2. Synthesestufe inerte organische Lösungsmittel infrage. Hierzu gehören vorzugsweise aromatische Kohlenwasserstoffe wie Benzol, Toluol oder Xylol, halogenierte Kohlenwasserstoffe wie Methylenchlorid, Chloroform, Tetrachlorkohlenstoff oder Chlorbenzol, Ketone wie Aceton oder Butanon, Ether wie Diethylether, Dioxan, Tetrahydrofuran, Formamide wie Dimethylformamid, Nitrile wie Acetonitril oder Propionitril, sowie die hochpolaren Lösungsmittel Dimethylsulfoxid oder Hexamethylphosphorsäuretriamid.

Die Umsetzung in der 2. Reaktionsstufe wird in Gegenwart von Säurebindern vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre

Alkylamine, Cycloalkylamine oder Arylalkylamine, wie beispielsweise Triethylamin, N,N-Dimethyl-benzylamin, weiterhin Pyridin sowie 1,4-Diazabicyclo(2,2,2)-octan und 1,5-Diazabicyclo(4,3,0)-non-5-en.

Die Reaktionstemperatur kann bei dem erfindungsgemäßen Verfahren innerhalb eines größeren Bereiches variiert werden. Im allgemeinen arbeitet man in der 1. Stufe zwischen 0 und 150°C, vorzugsweise bei der Siedetemperatur des verwendeten Verdünnungsmittels.

In der 2. Stufe arbeitet man im allgemeinen zwischen -20°C und +100°C, vorzugsweise zwischen 0°C und 60°C.

Das erfindungsgemäße Verfahren wird im allgemeinen in beiden Stufen bei Normaldruck durchgeführt.

Zur Durchführung des erfindungsgemäßen Verfahrens setzt man auf 1 Mol Cycloalkylendicarbonsäure der Formel (II) gewöhnlich 2 bis 3 Mol, vorzugsweise 2 bis 2,5 Mol Thionylchlorid (1. Stufe) und 2 bis 2,5 Mol, vorzugsweise 2 bis 2,2 Mol Jodpropargylalkohol (2. Stufe) ein.

Das Reaktionsgemisch wird mehrere Stunden bei der erforderlichen Temperatur gerührt. Die Isolierung der Endprodukte der Formel (I) erfolgt in allgemein üblicher Art und Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Venturia-Arten, wie z.B. gegen der Erreger des Apfelschorfes (Venturia inaequalis), zur Bekämpfung von Leptosphaeria-Arten, wie z.B. den Erreger der Braunfleckenkrankheit des Weizens (Leptosphaeria nodorum), zur Bekämpfung von Oomyceten, wie z.B. den Erreger der Kartoffel- oder Tomatenkraut- bzw. -knollenfäule (Phytophthora infestans), zur Bekämpfung von Mehltau- und Rostkrankheiten sowie Cochhiobolus an Getreide und zur Bekämpfung von Reiskrankheiten, wie z.B. Pellicularia sasakii, eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Fomulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele :

Beispiel 1 :

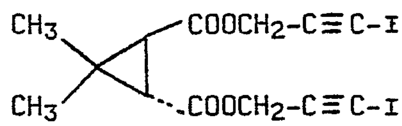

1. Stufe : 3,3-Dimethyl-cyclopropan-trans-1,2-dicarbonsäuredichlorid

Zu einer Lösung von 79 g (0,5 Mol) 3,3-Dimethyl-cyclopropan-trans-1,2-dicarbonsäure in 300 ml Tetrachlorkohlenstoff und 1 ml Dimethylformamid tropft man bei 40 bis 50°C 157g (1,32Mol) Thionylchlorid. Das Reaktionsgemisch wird 3 Stunden unter Rückfluß gekocht, dann destilliert man das Lösungsmittel im Vakuum ab. Der Rückstand wird im Vakuum destilliert. Man erhält so 92 g (95 % der Theorie) 3,3-Dimethyl-cyclopropan-trans-1,2-dicarbonsäuredichlorid in Form einer farblosen Flüssigkeit vom Siedepunkt 81 - 82°C/8 mm Hg.

2. Stufe : 3,3-Dimethyl-cyclopropan-trans-1,2-dicarbonsäure-di-(3-jodpropargyl)-ester

Eine Lösung von 7,4 g (0,038 Mol) 3,3-Dimethyl-cyclopropan-trans-1,2-dicarbonsäuredichlorid in 10 ml Toluol wird bei 20 bis 25°C zu einer Mischung aus 13,8 g (0,076 Mol) 3-Jodpropargylalkohol, 8 g (0,08 Mol) Triethylamin und 200 ml Toluol getropft. Das Reaktionsgemisch wird 18 Stunden bei Raumtemperatur gerührt und dann zweimal mit je 300 ml Wasser ausgeschüttelt. Die organische Phase wird über Natriumsulfat getrocknet; dann destilliert man das Lösungsmittel im Vakuum ab. Nach Zugabe von etwas Petroläther kann das

kristalline Produkt abgesaugt werden. Man erhält auf diese Weise 15 g (83 % der Theorie) 3,3-Dimethyl-cyclopropan-trans-1,2-dicarbonsäure-di-(3-jodpropargyl)-ester in Form farbloser Kristalle vom Schmelzpunkt 78°C.

Analog Beispiel (1) können die folgenden Verbindungen der Formel (I) hergestellt werden :

$$I-C\equiv C-CH_2-O-\underset{\underset{O}{\|}}{C}-A-\underset{\underset{O}{\|}}{C}-O-CH_2-C\equiv C-I \qquad (I)$$

| Beisp.-Nummer | | Schmelzpunkt |
|---|---|---|
| (2) | 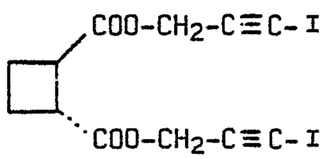 | 65°C |
| (3) | 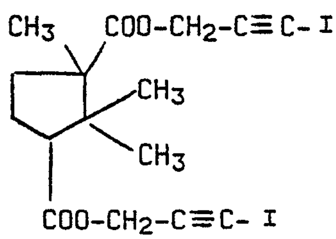 | 96°C |
| (4) | 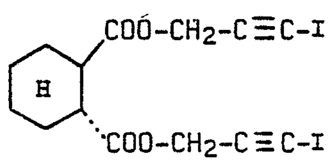 | 100°C |

Anwendungsbeispiele :

In den folgenden Anwendungsbeispielen werden die nachstehend angegebenen Verbindungen als Vergleichssubstanzen eingesetzt :

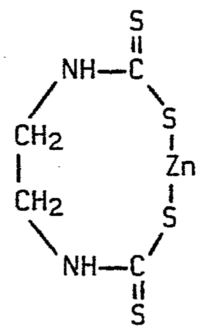

(A)

Zink-ethylen-1,2-bis-(dithiocarbamat)

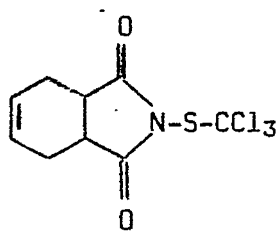

(B)

N-Trichlormethylthio-tetrahydrophthalimid

Beispiel A

Venturia-Test (Apfel) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß folgendem Herstellungsbeispiel : (1)

Beispiel B

Leptosphaeria nodorum-Test (Weizen) /protektiv

Lösungsmittel: 100 Gewichtsteile Dimethylformamid

Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Ueberlegeheit in der Wirksamkeit gegenüber dem Stand der Technik zeigen bei diesem Test z.B. die Verbindungen gemäß folgender Herstellungsbeispiele: 1, 2, 3 und 4.

Beispiel C

Phytophthora-Test (Tomate) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 2, 3 und 4.

Patentansprüche

1) Jodpropargylcarbonsäureester der allgemeinen Formel (I):

$$I-C\equiv C-CH_2-O-CO-A-CO-O-CH_2-C\equiv C-I \qquad (I)$$

in welcher

A für einen gegebenenfalls substituierten zweifach verknüpften Cycloalkylenrest steht.

2) Verbindungen der allgemeinen Formel (I) in Anspruch 1,

in welcher

A für einen 2-fach verknüpften Cycloalkylenrest mit 3 bis 7 Kohlenstoffatomen steht, welcher gegebenenfalls ein- bis dreifach, gleich oder verschieden durch Methyl, Ethyl und Chlor substituiert sein kann.

3) Verfahren zur Herstellung von Jodpropargylcarbonsäureestern der Formel (I):

$$I-C\equiv C-CH_2-O-CO-A-CO-O-CH_2-C\equiv C-I \qquad (I)$$

in welcher

A für einen gegebenenfalls substituierten zweifach verknüpften Cycloalkylenrest steht,

dadurch gekennzeichnet, daß man Cycloalkylendicarbonsäuren der allgemeinen Formel (II):

HO-CO-A-CO-OH (II)

in welcher

A die oben angegebene Bedeutung hat,

zunächst mit Thionylchlorid, gegebenenfalls in Gegenwart eines Verdünnungsmittels, umsetzt und die so erhaltenen Cycloalkylendicarbonsäuredichloride der allgemeinen Formel (III):

Cl-CO-A-CO-Cl (III)

in welcher

A die oben angegebene Bedeutung hat,

in Gegenwart eines Säurebindemittels und gegebenenfalls in Gegenwart eines Verdünnungsmittels mit Jodpropargylalkohol umsetzt.

4) Pflanzenschutzmittel, gekennzeichnet durch einen Gehalt an mindestens einem Jodpropargylcarbonsäureester der Formel (I) in Ansprüchen 1 und 3.

5) Fungizide Mittel, gekennzeichnet durch einen Gehalt an mindestens einem Jodpropargylcarbonsäureester der Formel (I) in Ansprüchen 1 und 3.

6) Verfahren zur Bekämpfung von Pilzen, dadurch gekennzeichnet, daß man Jodpropargylcarbonsäureester der Formel (I) in Ansprüchen 1 und 3 auf Pilze oder ihren Lebensraum einwirken läßt.

7) Verwendung von Jodpropargylcarbonsäureestern der Formel (I) in Ansprüchen 1 und 3 zur Bekämpfung von Pilzen.

8) Verfahren zur Herstellung von fungiziden Mitteln, dadurch gekennzeichnet, daß man Jodpropargylcarbonsäureester der Formel (I) in Ansprüchen 1 und 3 mit Streckmitteln und/oder oberflächenaktiven Mitteln vermischt.